(19) Europäisches Patentamt
European Patent Office
Office européen des brevets

(11) **EP 4 064 961 B1**

(12) **FASCICULE DE BREVET EUROPEEN**

(45) Date de publication et mention de la délivrance du brevet:
**07.08.2024 Bulletin 2024/32**

(21) Numéro de dépôt: **20808442.6**

(22) Date de dépôt: **24.11.2020**

(51) Classification Internationale des Brevets (IPC):
*A61B 5/00* (2006.01)   *G16H 40/63* (2018.01)
*A61B 5/145* (2006.01)   *A61B 5/053* (2021.01)
*G16H 40/40* (2018.01)   *G16H 50/20* (2018.01)
*G16H 10/40* (2018.01)   *A61B 5/0531* (2021.01)

(52) Classification Coopérative des Brevets (CPC):
**A61B 5/14517; A61B 5/4266; G16H 10/40;**
**G16H 40/63; G16H 50/20;** A61B 5/0004;
A61B 5/0531; A61B 5/14546; A61B 5/6802

(86) Numéro de dépôt international:
**PCT/EP2020/083210**

(87) Numéro de publication internationale:
**WO 2021/105122 (03.06.2021 Gazette 2021/22)**

(54) **DETECTION D'UNE ESPECE CHIMIQUE DANS LA SUEUR D'UN SUJET**

DETEKTION EINER CHEMISCHEN SPEZIES IM SCHWEISS EINES PROBANDEN

DETECTION OF A CHEMICAL SPECIES IN THE SWEAT OF A SUBJECT

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**
Etats de validation désignés:
**MA TN**

(30) Priorité: **28.11.2019 FR 1913400**

(43) Date de publication de la demande:
**05.10.2022 Bulletin 2022/40**

(73) Titulaire: **NOPTRACK**
**81860 Castres (FR)**

(72) Inventeurs:
• **RIVIERE, Philippe**
**31500 Toulouse (FR)**
• **AMATORE, Christian**
**75013 Paris (FR)**
• **FAVRE, Gilles**
**31270 Cugnaux (FR)**
• **LABRUNÉE, Marc**
**31400 Toulouse (FR)**
• **DAUMAS, Frédéric**
**31120 Pinsaguel (FR)**

(74) Mandataire: **Loyer & Abello**
**9, rue Anatole de la Forge**
**75017 Paris (FR)**

(56) Documents cités:
**EP-A1- 0 384 090**          **WO-A1-2019/170776**
**WO-A1-2019/210240**   **WO-A1-2019/229380**
**WO-A2-2016/061362**   **WO-A2-98/08480**
**US-A1- 2018 070 869**   **US-A1- 2019 008 448**
**US-A1- 2019 110 722**

• **LIN-MEI LI ET AL: "Vascular lumen simulation and highly-sensitive nitric oxide detection using three-dimensional gelatin chip coupled to TiC/C nanowire arrays microelectrode", LAB ON A CHIP, vol. 12, no. 21, 1 January 2012 (2012-01-01), UK, pages 4249, XP055526526, ISSN: 1473-0197, DOI: 10.1039/c2lc40148g**
• **MICHAEL D. PLUTH ET AL: "Biochemistry of Mobile Zinc and Nitric Oxide Revealed by Fluorescent Sensors", ANNUAL REVIEW OF BIOCHEMISTRY, vol. 80, no. 1, 7 July 2011 (2011-07-07), US, pages 333 - 355, XP055525052, ISSN: 0066-4154, DOI: 10.1146/annurev-biochem-061009-091643**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**(Cont. page suivante)**

- KENNY WALTER: "Novel Wearable Sensor Monitors Health Through Sweat Using Nanotech", -RESEARCH & DEVELOPMENT WORLD, 26 April 2019 (2019-04-26), XP055738166, Retrieved from the Internet <URL:https://www.rdworldonline.com/novel-wearable-sensor-monitors-health-through-sweat-using-nanotech/> [retrieved on 20201008]
- RICHARD WELLER ET AL: "Nitric Oxide Is Generated on the Skin Surface by Reduction of Sweat Nitrate", JOURNAL OF INVESTIGATIVE DERMATOLOGY, vol. 107, no. 3, 1 September 1996 (1996-09-01), pages 327 - 331, XP055205544, DOI: 10.1111/1523-1747.ep12363167
- BRUCH-GERHARZ D ET AL: "Nitric oxide in human skin: current status and future prospects", JOURNAL OF INVESTIGATIVE DERMATOLOGY, ELSEVIER, NL, vol. 110, no. 1, 1 January 1998 (1998-01-01), pages 1 - 7, XP002254968, ISSN: 0022-202X, DOI: 10.1046/J.1523-1747.1998.00084.X
- BYEONG WAN AN ET AL: "Smart Sensor Systems for Wearable Electronic Devices", POLYMERS, vol. 9, no. 12, 25 July 2017 (2017-07-25), pages 303, XP055526164, DOI: 10.3390/polym9080303
- SALZITSA ANASTASOVA ET AL: "A wearable multisensing patch for continuous sweat monitoring", BIOSENSORS AND BIOELECTRONICS, vol. 93, 1 July 2017 (2017-07-01), AMSTERDAM, NL, pages 139 - 145, XP055482880, ISSN: 0956-5663, DOI: 10.1016/j.bios.2016.09.038
- A. ABELLÁN-LLOBREGAT ET AL: "A stretchable and screen-printed electrochemical sensor for glucose determination in human perspiration", BIOSENSORS AND BIOELECTRONICS, vol. 91, 1 May 2017 (2017-05-01), AMSTERDAM, NL, pages 885 - 891, XP055738168, ISSN: 0956-5663, DOI: 10.1016/j.bios.2017.01.058
- JUNGIL CHOI ET AL: "Skin-interfaced systems for sweat collection and analytics", SCIENCE, vol. 4, no. 2, 16 February 2018 (2018-02-16), pages eaar3921, XP055526287, ISSN: 0036-8075, DOI: 10.1126/sciadv.aar3921
- YEJIN HA ET AL: "Measurements of Location-Dependent Nitric Oxide Levels on Skin Surface in relation to Acupuncture Point", EVIDENCE-BASED COMPLEMENTARY AND ALTERNATIVE MEDICINE, vol. 2012, 1 January 2012 (2012-01-01), pages 1 - 7, XP055524807, ISSN: 1741-427X, DOI: 10.1155/2012/781460
- ALBERT MAAREK ET AL: "Detection of neuropathy using a sudomotor test in type 2 diabetes", DEGENERATIVE NEUROLOGICAL AND NEUROMUSCULAR DISEASE, 9 January 2015 (2015-01-09), pages 1, XP055525495, ISSN: 1179-9900, DOI: 10.2147/DNND.S75857
- YOUNGMI LEE ET AL: "Improved Planar Amperometric Nitric Oxide Sensor Based on Platinized Platinum Anode. 1. Experimental Results and Theory When Applied for Monitoring NO Release from Diazeniumdiolate-Doped Polymeric Films - Analytical Chemistry (ACS Publications)", 1 January 2004 (2004-01-01), XP055526149, Retrieved from the Internet <URL:https://pubs.acs.org/doi/10.1021/ac035064h> [retrieved on 20181122]
- MA ET AL: "Evidence of enhanced non-enzymatic generation of nitric oxide on the skin surface of acupuncture points: An innovative approach in humans", NITRIC OXIDE: BIOLOGY AND CHEMISTRY, ACADEMIC PRESS, AMSTERDAM, NL, vol. 17, no. 2, 18 August 2007 (2007-08-18), pages 60 - 68, XP022207173, ISSN: 1089-8603, DOI: 10.1016/J.NIOX.2007.05.004
- MALLIKA BARIYA ET AL: "Wearable sweat sensors", NATURE ELECTRONICS, vol. 1, no. 3, 1 March 2018 (2018-03-01), pages 160 - 171, XP055525597, ISSN: 2520-1131, DOI: 10.1038/s41928-018-0043-y
- JOHN E LEWIS ET AL: "New method of sudomotor function measurement to detect microvascular disease and sweat gland nerve or unmyelinated C fiber dysfunction in adults with retinopathy", JOURNAL OF DIABETES & METABOLIC DISORDERS, BIOMED CENTRAL LTD, LONDON, UK, vol. 16, no. 1, 12 June 2017 (2017-06-12), pages 1 - 10, XP021245874, DOI: 10.1186/S40200-017-0307-5
- YEJIN HA ET AL: "Insertable NO/CO Microsensors Recording Gaseous Vasomodulators Reflecting Differential Neuronal Activation Level with Respect to Seizure Focus", ACS CHEMICAL NEUROSCIENCE, vol. 8, no. 9, 5 July 2017 (2017-07-05), US, pages 1853 - 1858, XP055524923, ISSN: 1948-7193, DOI: 10.1021/acschemneuro.7b00141
- KICHANG LEE ET AL: "Role of nitric oxide in methacholine-induced sweating and vasodilation in human skin", JOURNAL OF APPLIED PHYSIOLOGY, 1 April 2006 (2006-04-01), United States, pages 1355 - 1360, XP055525486, Retrieved from the Internet <URL:https://pdfs.semanticscholar.org/c834/1f4a660265a12e6e0bf87ba563ab9b9fe1ce.pdf> DOI: 10.1152/japplphysiol.00122.2005
- VINCENZO F. CURTO ET AL: "Real-time sweat pH monitoring based on a wearable chemical barcode micro-fluidic platform incorporating ionic liquids", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 171-172, 1 August 2012 (2012-08-01), NL, pages 1327 - 1334, XP055451404, ISSN: 0925-4005, DOI: 10.1016/j.snb.2012.06.048

- **HNIN YIN YIN NYEIN ET AL: "A Wearable Microfluidic Sensing Patch for Dynamic Sweat Secretion Analysis", ACS SENSORS, vol. 3, no. 5, 9 May 2018 (2018-05-09), pages 944 - 952, XP055525587, ISSN: 2379-3694, DOI: 10.1021/acssensors.7b00961**
- **KRAMER RYAN M ET AL: "Analytical determination and detection of individual odor signatures", SENSING TECHNOLOGIES FOR GLOBAL HEALTH, MILITARY MEDICINE, DISASTER RESPONSE, AND ENVIRONMENTAL MONITORING II; AND BIOMETRIC TECHNOLOGY FOR HUMAN IDENTIFICATION IX, SPIE, 1000 20TH ST. BELLINGHAM WA 98225-6705 USA, vol. 8371, no. 1, 11 May 2012 (2012-05-11), pages 1 - 14, XP060002731, DOI: 10.1117/12.919921**
- **YEJIN HA ET AL: "Amperometric Microsensors Monitoring Glutamate-Evoked In Situ Responses of Nitric Oxide and Carbon Monoxide from Live Human Neuroblastoma Cells", SENSORS, vol. 17, no. 7, 19 July 2017 (2017-07-19), pages 1661, XP055524813, DOI: 10.3390/s17071661**
- **GF CLOUGH ET AL: "Measurement of nitric oxide concentration in human skin in vivo using dermal microdialysis", EXPERIMENTAL PHYSIOLOGY, vol. 83, no. 3, 1 May 1998 (1998-05-01), GB, pages 431 - 434, XP055526121, ISSN: 0958-0670, DOI: 10.1113/expphysiol.1998.sp004126**
- **LUO XIAOJIN ET AL: "A Wearable Amperometric Biosensor on a Cotton Fabric for Lactate", IEEE ELECTRON DEVICE LETTERS, IEEE SERVICE CENTER, NEW YORK, NY, US, vol. 39, no. 1, 1 January 2018 (2018-01-01), pages 123 - 126, XP011675011, ISSN: 0741-3106, [retrieved on 20171226], DOI: 10.1109/LED.2017.2777474**

## Description

### Domaine technique

L'invention concerne des procédés et appareils pour détecter au moins une espèce chimique dans la sueur d'un sujet humain ou animal, ladite au moins une espèce chimique dissoute incluant le monoxyde d'azote NO.

### Arrière-plan technologique

Le monoxyde d'azote est un gaz qui constitue un messager intercellulaire. Il est un important messager cardiovasculaire du stress par mécanotransduction. Il est émis en particulier afin stimuler la vasodilatation du système vasculaire lors d'efforts musculaires. Les variations de son flux de production dans le sang et donc dans les liquides en équilibre avec le sang, par exemple la sueur, constituent donc un indicateur particulièrement pertinent de la capacité cardiovasculaire d'un patient à s'adapter à la puissance musculaire demandée lors de tests d'effort.

Dans le cas d'une maladie cardio-vasculaire, les dispositifs actuellement existants et les outils de prévention et de prédiction sont soit limités à une mesure indirecte du monoxyde d'azote du patient au repos, soit limités à une mesure directe différée de plusieurs heures par rapport à une observation d'un problème pathologique. Dans tous les cas, les mesures ne peuvent être réalisées que dans un environnement médicalisé.

### Résumé

Certains aspects de l'invention sont basés sur l'idée que la mesure quantitative des variations de la concentration de monoxyde d'azote dans la sueur fournit une méthode non invasive de surveillance de la capacité cardiovasculaire lors de contrôles préventifs ou afin d'établir un diagnostic.

Certains aspects de l'invention sont basés sur le constat que, en présence de dioxygène, le monoxyde d'azote réagit pour produire un ion nitrite (NO2-) via une réaction de stoechiométrie globale 1:1. En d'autres termes, tandis que les variations de la concentration de monoxyde d'azote représentent l'état présent des réponses du système cardiovasculaire à un effort donné, les variations de la concentration de l'ion nitrite constituent une archive temporelle de ces réponses.

Certains aspects sont basés sur l'idée que les variations de concentrations du monoxyde d'azote et de l'ion nitrite peuvent être détectées et quantifiées de manière couplée par un dispositif électrochimique intégré.

Certains aspects sont basés sur le constat que le monoxyde d'azote est produit par des enzymes spécialisées (les NO-synthases) à partir de la dégradation de L-arginine intracellulaire en présence de dioxygène ($O_2$) et d'une source d'électrons. Lorsque la disponibilité en L-arginine diminue du fait d'une forte consommation (par exemple suite à un effort prolongé) ou d'une carence chronique, les NO-synthases continuent à réagir avec l'oxygène en se bornant à réduire le dioxygène en ion superoxyde ($O_2^-$). Ce dernier évolue spontanément très rapidement en peroxyde d'hydrogène ($H_2O_2$), via une réaction de stoechiométrie globale 2:1. Certains aspects de l'invention sont basés sur l'idée que la présence de concentrations détectables de peroxyde d'hydrogène dans le sang, et donc dans la sueur, fournit un indicateur représentant un niveau de souffrance du réseau cardiovasculaire. De plus, en présence de sels métalliques, peroxyde d'hydrogène se décompose en espèces radicalaires très toxiques ($HO\bullet$, $HO_2\bullet$, etc.) susceptibles d'induire des dégâts très importants sur les cellules du système cardiovasculaire y compris celles du coeur. Certains aspects de l'invention sont basés sur l'idée que la détection d'une production de peroxyde d'hydrogène en parallèle de la détection d'une production de monoxyde d'azote et/ou d'ion nitrite est pertinente pour évaluer les capacités cardiovasculaires d'un patient.

Certains aspects de l'invention sont basés sur l'idée de détecter les trois espèces chimiques précitées, à savoir le peroxyde d'hydrogène, le monoxyde d'azote et l'ion nitrite, de manière couplée par un dispositif électrochimique intégré.

Certains aspects sont basés sur le constat que le système physiologique d'un sujet est dynamique, puisque le débit volumique de sueur peut varier pour ajuster la capacité d'élimination des calories produites en fonction de la puissance musculaire délivrée. Les flux d'échange de chaque espèce chimique au niveau des interfaces entre le sang et la sueur peuvent varient en fonction d'un effort fourni par le sujet. Certains aspects de l'invention sont basés sur l'idée de détecter de manière quantitative et dynamique la production d'une ou plusieurs des espèces chimiques précitées par le système cardiovasculaire, par exemple lors d'un test d'effort ou d'un suivi médical du sujet.

Pour cela, l'invention fournit un appareil de détection tel que défini dans la revendication 1.

On entend par une espèce chimique dans un liquide biologique que l'espèce chimique, notamment le monoxyde d'azote, est dissoute dans le liquide biologique.

On entend par un épiderme le tissu végétal superficiel formant une couche protectrice des parties aériennes d'un végétal ou la couche superficielle de la peau chez l'homme et les animaux. Le liquide biologique est par exemple l'exsudat chez les végétaux ou la sueur chez l'homme et les animaux.

Selon des modes de réalisation, un tel appareil peut comporter une ou plusieurs des caractéristiques suivantes.

Selon un mode de réalisation, le ou chaque ou au moins un dit capteur électrochimique est configuré pour produire en outre un signal représentatif d'une vitesse d'écoulement du flux de sueur ou d'un débit volumique de sueur.

Selon un mode de réalisation, le dispositif détecteur est configuré pour produire un signal représentatif d'une production instantanée de ladite au moins une espèce chimique dans la zone d'investigation sur la base du signal représentatif de la concentration de ladite au moins une espèce chimique et du signal représentatif de la vitesse d'écoulement du flux de sueur.

Par « production instantanée », on désigne une mesure prise sur une durée très faible par rapport au temps caractéristique de la variation de réponse physiologique du sujet. Ce temps caractéristique est typiquement de l'ordre de une à plusieurs minutes pour un sujet humain.

Selon un mode de réalisation, le ou chaque ou au moins un dit capteur électrochimique comporte au moins une électrode de travail et une contre-électrode disposées dans le circuit fluidique, le capteur électrochimique étant configuré pour produire un signal représentatif de la concentration de la au moins une espèce chimique par une mesure électrique, notamment ampérométrique, entre la au moins une électrode de travail et la contre-électrode.

Selon un mode de réalisation, le ou chaque ou au moins un dit capteur électrochimique comporte une électrode de travail amont et une électrode de travail aval espacées dans le circuit fluidique dans une direction d'écoulement du flux de sueur, le capteur électrochimique étant configuré pour produire le signal représentatif de la vitesse d'écoulement par mesure d'un retard entre une variation de courant ou de potentiel dans l'électrode de travail amont et une variation de courant ou de potentiel dans l'électrode de travail aval.

Selon un mode de réalisation, la distance L séparant l'électrode de travail amont et l'électrode de travail aval est comprise entre 0,05 mm et 1 cm.

Selon un mode de réalisation, le ou au moins un dit capteur électrochimique est configuré pour polariser les électrodes à un potentiel électrique d'oxydation du monoxyde d'azote et le dispositif détecteur comporte un filtre configuré pour filtrer le flux de sueur atteignant le capteur électrochimique afin d'éliminer notamment le peroxyde d'hydrogène.

Un tel filtre peut être réalisé de différentes manières, par exemple avec une membrane en polytétrafluoroéthylène (PTFE) ou une membrane de type eugénol, qui peut être disposée dans le circuit fluidique. Selon un mode de réalisation, le filtre comporte une couche d'eugénol (4-allyl-2méthoxyphénol) déposée sur au moins une électrode du capteur électrochimique.

Cette configuration fondée sur une électrode détectrice modifiée avec ce type de filtre spécifique à la mesure de NO produit directement un signal représentatif de la concentration de monoxyde d'azote NO sur la base du signal de mesure ampérométrique.

Selon des modes de réalisation décrits ci-dessous, le dispositif détecteur est réalisé de manière à pouvoir détecter simultanément ou séquentiellement deux ou trois des espèces chimiques précitées, avec un ou plusieurs capteurs électrochimiques.

Selon un mode de réalisation, ladite au moins une espèce chimique comprend le monoxyde d'azote NO et l'ion nitrite $NO_2^-$. Selon un mode de réalisation, ladite au moins une espèce chimique comprend le monoxyde d'azote NO et le peroxyde d'hydrogène $H_2O_2$. Selon un de réalisation, ladite au moins une espèce chimique comprend le monoxyde d'azote NO, l'ion nitrite $NO_2^-$ et le peroxyde d'hydrogène $H_2O_2$.

Selon un mode de réalisation permettant une détection séquentielle, le ou chaque ou au moins un dit capteur électrochimique est configuré pour détecter séquentiellement plusieurs espèces chimiques au cours d'une pluralité d'étapes de mesure, le capteur électrochimique étant configuré pour polariser les électrodes à un potentiel électrique d'oxydation du peroxyde d'hydrogène $H_2O_2$ au cours d'une première étape et polariser les électrodes à un potentiel électrique d'oxydation du monoxyde d'azote NO au cours d'une deuxième étape, et le dispositif détecteur est configuré pour produire un signal représentatif d'une concentration de monoxyde d'azote NO sur la base d'un premier signal de mesure ampérométrique obtenu lors de la première étape et d'un deuxième signal de mesure ampérométrique obtenu lors de la deuxième étape.

Avantageusement dans ce cas, le ou chaque ou au moins un dit capteur électrochimique est configuré pour polariser les électrodes à un potentiel électrique d'oxydation de l'ion nitrite $NO_2^-$ au cours d'une troisième étape, et le dispositif détecteur est configuré pour produire un signal représentatif d'une concentration de l'ion nitrite $NO_2^-$ sur la base desdits premier et deuxième signaux de mesure ampérométriques et d'un troisième signal de mesure ampérométrique obtenu lors de la troisième étape.

Selon un mode de réalisation, le dispositif détecteur est réalisé de manière à pouvoir détecter séquentiellement trois des espèces chimiques précitées, avec un seul capteur électrochimique au cours d'une pluralité d'étapes de séquences temporelles de mesures. Selon ce mode de réalisation, lors d'une séquence donnée, le capteur électrochimique est configuré pour polariser une électrode en platine platiné (noir de platine) séquentiellement au potentiel électrochimique d'oxydation du peroxyde d'hydrogène $H_2O_2$ au cours d'une première étape temporelle de quelques secondes (5 s par exemple), puis à celui de l'oxydation du monoxyde d'azote NO au cours d'une deuxième étape temporelle de même durée, et éventuellement à celui de l'ion nitrite $NO_2^-$ lors d'une troisième étape temporelle de même durée. Le dispositif détecteur est configuré pour produire un signal représentatif d'une concentration de monoxyde d'azote NO sur la base d'un premier signal de mesure ampérométrique obtenu lors de la première étape et d'un deuxième signal de mesure ampérométrique obtenu lors de la deuxième étape. Cette séquence se répète autant que nécessaire pendant la durée totale du test d'effort. La résolution d'un système de trois équations (les courants mesurés séquentiellement sur l'électrode polarisée à chaque potentiel dans une séquence) à deux ou trois inconnues (les concentrations en $H_2O_2$, NO et $NO_2$-) fournit les valeurs de chacune des trois concentrations au moment où chaque séquence est réalisée sur la base des trois mesures.

Selon un autre mode de réalisation permettant une détection simultanée, le dispositif détecteur comporte :

un premier circuit fluidique couplé à l'élément collecteur pour conduire un premier flux de sueur provenant de la zone d'investigation et un premier capteur électrochimique comportant des électrodes disposées dans le premier circuit fluidique, le premier capteur électrochimique étant configuré pour polariser les électrodes à un potentiel électrique d'oxydation du peroxyde d'hydrogène $H_2O_2$, et

un deuxième circuit fluidique couplé à l'élément collecteur pour conduire un deuxième flux de sueur provenant de la zone d'investigation et un deuxième capteur électrochimique comportant des électrodes disposées dans le deuxième circuit fluidique, le deuxième capteur électrochimique étant configuré pour polariser les électrodes à un potentiel électrique d'oxydation du monoxyde d'azote NO,

et le dispositif détecteur est configuré pour produire un signal représentatif d'une concentration de monoxyde d'azote NO sur la base d'un premier signal de mesure ampérométrique produit par le premier capteur électrochimique et d'un deuxième signal de mesure ampérométrique produit par le deuxième capteur électrochimique.

Avantageusement dans ce cas, le dispositif détecteur comporte en outre :
un troisième circuit fluidique couplé à l'élément collecteur pour conduire un troisième flux de sueur provenant de la zone d'investigation et un troisième capteur électrochimique comportant des électrodes disposées dans le troisième circuit fluidique, le troisième capteur électrochimique étant configuré pour polariser les électrodes à un potentiel électrique d'oxydation de l'ion nitrite $NO_2$-, et le dispositif détecteur est configuré pour produire un signal représentatif d'une concentration de l'ion nitrite $NO_2$- sur la base desdits premier et deuxième signaux de mesure ampérométriques et d'un troisième signal de mesure ampérométrique produit par le troisième capteur électrochimique.

Selon ce mode de réalisation, le dispositif détecteur comporte trois circuits microfluidiques parallèles alimentés en parallèle par le même élément collecteur de sueur.

Selon un mode de réalisation, le dispositif détecteur comporte en outre :
un autre circuit fluidique, par exemple un quatrième circuit fluidique, couplé à l'élément collecteur pour conduire un autre flux de sueur provenant de la zone d'investigation, par exemple quatrième flux de sueur, et un autre capteur électrochimique, par exemple quatrième capteur électrochimique, comportant des électrodes disposées dans le quatrième circuit fluidique.

Selon ce mode de réalisation, ledit autre ou quatrième capteur électrochimique est configuré pour polariser les électrodes à un potentiel électrique d'oxydation du monoxyde d'azote et le quatrième circuit fluidique comporte un filtre configuré pour filtrer le flux de sueur atteignant le quatrième capteur électrochimique afin d'éliminer notamment le peroxyde d'hydrogène.

Selon un mode de réalisation, l'élément collecteur comporte un corps fibreux pour conduire le liquide biologique de la zone d'investigation, notamment la sueur, par capillarité. Un tel corps fibreux peut être un matériau tissé ou non tissé.

Selon un mode de réalisation, l'appareil comporte en outre une enveloppe entourant l'élément collecteur de manière à former une barrière étanche autour de la zone d'investigation par contact avec l'épiderme dudit sujet.

Grâce à ces caractéristiques, les gaz, les liquides et les micro-organismes tels que bactéries ou virus, situés à l'extérieur de la zone d'investigation ne peuvent pas entrer dans la zone d'investigation. L'étanchéité du contact entre l'enveloppe et l'épiderme assure que l'espèce chimique détectée provient du liquide biologique produit par la zone d'investigation, et non d'un flux venant de l'extérieur.

Selon un mode de réalisation, l'appareil comporte en outre un dispositif de communication filaire ou sans fil configuré pour transmettre un ou plusieurs signaux de mesure produits par le dispositif détecteur à destination d'un appareil de post-traitement.

Selon un mode de réalisation, le ou chaque ou au moins un dit circuit fluidique est formé dans un support isolant et les électrodes du ou chaque ou d'au moins un dit capteur électrochimique sont constituées de dépôts métalliques sur ledit support isolant. Un tel support isolant peut être réalisé dans un matériau choisi parmi les élastomères, par exemple parmi le polydiméthylsiloxane (PDMS), les polyimides, les résines époxydes et le parylène.

Selon un mode de réalisation, les dépôts métalliques sont sélectionnés dans le groupe consistant en l'argent (Ag), l'or (Au), le platine (Pt) et le noir de platine. Selon un mode de réalisation, le capteur électrochimique comporte une électrode de référence en chlorure d'argent (AgCl).

Dans une réalisation intéressante, des électrodes sont constituées de dépôts de graphène dopé par des nanoparticules d'argent (Ag) ou d'or (Au), les nanoparticules étant fonctionnalisées par des liants du monoxyde d'azote, notamment la guanylyl-cyclase ou des porphyrines.

Selon un mode de réalisation, l'appareil est configuré pour effectuer et transmettre périodiquement des mesures, par exemple à une fréquence paramétrable ou à une fréquence dépendant d'un état d'activité détecté par l'appareil. Par exemple, l'appareil peut comporter un module gyroscopique et/ou d'un accéléromètre pour détecter l'état d'activité du sujet. Ainsi, il est possible de détecter l'état d'activité du sujet pendant les analyses de la sueur, afin de faciliter une analyse des corrélations entre l'état d'activité du sujet et la production des espèces chimiques analysées.

Selon un mode de réalisation, l'appareil comprend un module de géolocalisation.

Selon un mode de réalisation, l'invention fournit également un procédé selon la revendication 20.

Les mesures de la production d'une ou plusieurs des espèces chimiques précitées par le sujet peuvent être exploitée dans diverses applications, par exemple pour évaluer une souffrance des tissus vasculaires du sujet à partir de ces mesures ou pour évaluer une capacité cardiovasculaire du sujet à partir de ces mesures.

D'autres applications possibles sont le diagnostic, la prise en charge thérapeutique et le suivi de pathologies telles que maladie cardio-vasculaire, maladie neurodégénérative, hypertension artérielle pulmonaire, cancer, hyper-cholestérolémie, diabète, dysfonctionnement du système endothélial, artériosclérose, pathologie thrombotique ou ischémique, dysfonctionnement de l'inhibition d'accumulation plaquettaire ou de l'adhérence leucocytaire ou de la prolifération de cellules des fibres musculaires lisses, inflammation bronchique, asthme, maladie d'Alzheimer.

D'autres applications possibles sont la surveillance de la croissance et/ou de la souffrance musculaire d'une personne, par exemple une personne soumise à un entrainement physique, la prévention des blessures dues au surentrainement et/ou l'augmentation des performances musculaires du sujet.

**Brève description des figures**

Pour mieux faire comprendre l'objet de l'invention, on va en décrire ci-après, à titre d'exemple purement démonstratif et non limitatif, un mode de réalisation représenté sur le dessin annexé. Sur ce dessin :

[Fig. 1] La figure 1 représente une vue schématique d'un sujet vu de dos sur lequel a été mis en place un appareil selon un mode de réalisation,

[Fig. 2] La figure 2 est une vue en perspective représentant un appareil de détection selon un mode de réalisation,

[Fig. 3] La figure 3 représente une vue éclatée de l'appareil de la figure 2,

[Fig. 4] la figure 4 est une vue schématique en coupe d'un élément collecteur selon un premier mode de réalisation pouvant être utilisé dans l'appareil de la figure 2,

[Fig. 5] la figure 5 est une vue schématique en coupe d'un élément collecteur selon un deuxième mode de réalisation pouvant être utilisé dans l'appareil de la figure 2,

[Fig. 6] la figure 6 est une représentation schématique fonctionnelle d'un circuit fluidique pouvant être utilisé dans l'appareil de la figure 2,

[Fig. 7] la figure 7 est une représentation schématique en perspective d'un capteur électrochimique pouvant être utilisé dans le circuit fluidique de la figure 6,

[Fig. 8] la figure 8 est un chronogramme illustrant un procédé de détection pouvant être effectué avec le capteur électrochimique de la figure 7,

[Fig. 9] la figure 9 est une représentation schématique fonctionnelle d'un dispositif détecteur pouvant être utilisé dans l'appareil de la figure 2,

[Fig. 10] la figure 10 est un diagramme d'étapes illustrant un procédé pouvant être mis en oeuvre avec l'appareil de la figure 2,

[Fig. 11] la figure 11 est un graphique illustrant un résultat des mesures pouvant être obtenues avec l'appareil de la figure 2.

## Description des modes de réalisation

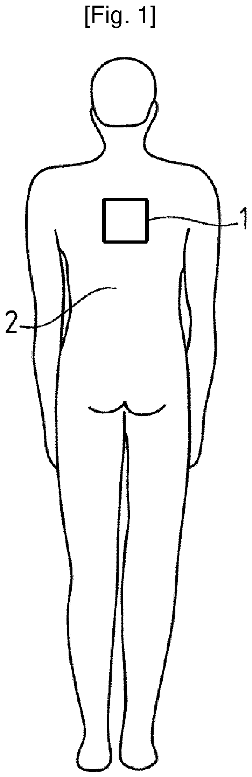

La figure 1 représente un appareil de détection 1 disposé sur la peau d'un sujet humain 2, par exemple dans le dos du sujet, et destiné à réaliser des mesures quantitatives d'espèces chimiques dissoutes dans la sueur, dont le monoxyde d'azote dissous dans la sueur, et éventuellement l'ion nitrite et le peroxyde d'hydrogène.

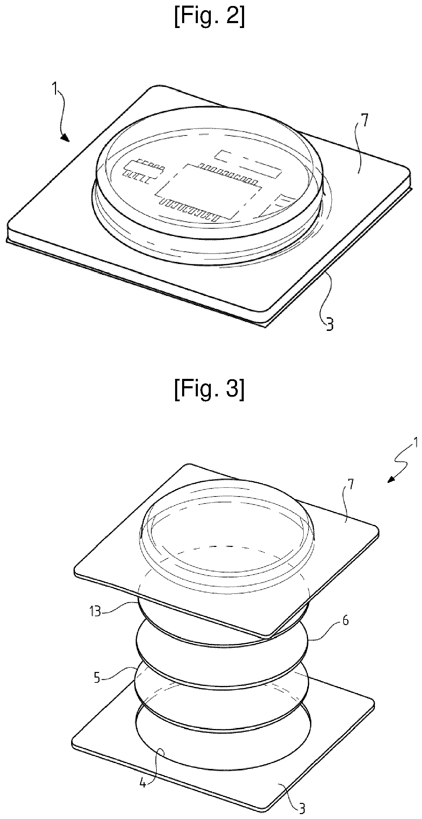

En référence à la figure 2, l'appareil de détection 1 se présente par exemple sous la forme d'un boitier peu encombrant qui comporte une embase de fixation 3 faite d'un matériau souple biocompatible, de préférence autocollant, que l'on peut positionner directement sur la peau du sujet et une enveloppe supérieure 7, de préférence étanche à l'eau, par exemple en silicone, qui renferme les autres composants de l'appareil de détection 1.

En référence à la figure 3, une partie centrale de l'embase 3 comporte un évidement circulaire 4 délimitant une zone d'investigation sur la peau du sujet, faisant par exemple quelques mm à quelques cm de diamètre. L'évidement circulaire 4 contient un élément collecteur 5, qui est ainsi positionné directement sur la peau 2 du sujet lorsque l'appareil est en fonctionnement. L'évidement 4 peut prendre une autre forme, par exemple une ellipse, un triangle, un rectangle, un carré, un polygone ou autre.

L'élément collecteur 5 comprend par exemple un corps fibreux, tel que du coton ou un matériau non tissé. L'élément collecteur 5 est relié à un support 6 portant un ou plusieurs circuits fluidiques et un ou plusieurs capteurs comme il sera décrit plus bas. Le support 6 peut être un support rigide ou flexible, par exemple en polyimide. L'élément collecteur 5 remplit la fonction d'amener la sueur produite dans la zone d'investigation jusqu'aux circuits fluidiques du support 5 pour permettre la détection d'une ou plusieurs espèces chimiques à l'aide des capteurs. Pour cela, l'élément collecteur 5 peut être disposé de différentes manières.

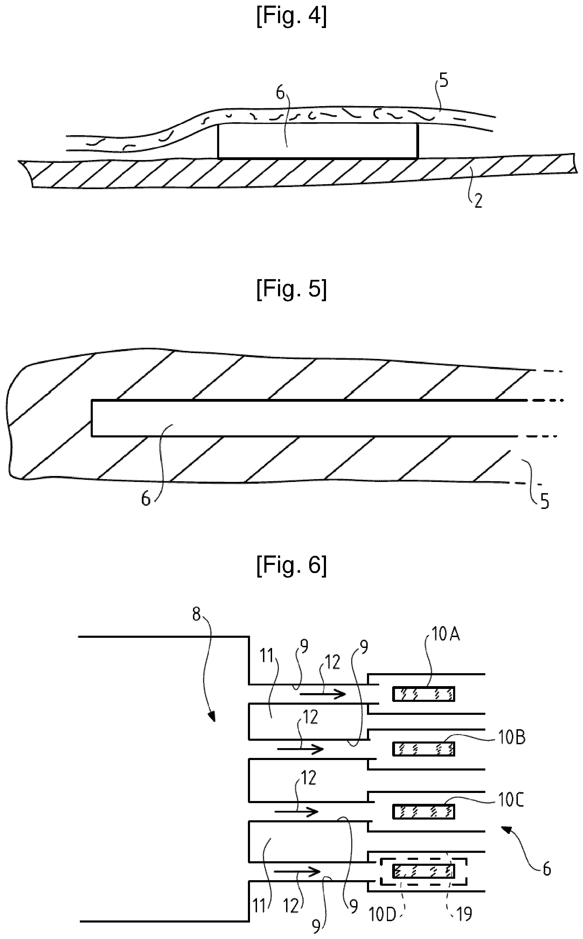

Dans l'exemple de la figure 4, l'élément collecteur 5 présente une partie en contact avec la peau 2 à côté du support 6 et une partie venant recouvrir une face supérieure du support 6. En d'autres termes, l'élément collecteur 5 est à cheval sur la peau et le support 6. Sur la face supérieure du support 6, l'élément collecteur 5 communique avec les circuits fluidiques pourvus des capteurs.

Dans l'exemple de la figure 5, l'élément collecteur 5 vient prendre en sandwich le support 6. Il en résulte qu'une partie de l'élément collecteur 5 disposée sous le support 6 est disposée contre la peau. Une partie de l'élément collecteur 5 prolongeant la partie disposée sur la peau est rabattue sur le support 6 venant ainsi recouvrir le support 6. Sur la face supérieure du support 6, l'élément collecteur 5 communique avec les circuits fluidiques pourvus des capteurs.

En référence à la figure 6, le support 6 porte un système microfluidique 8 alimenté par capillarité par l'élément collecteur 5. La sueur est collectée par la partie de l'élément collecteur 5 placée en contact avec la peau 2 puis transférée par capillarité afin de circuler dans le système microfluidique 8. Le système microfluidique 8 peut comporter un ou plusieurs circuits fluidiques, à savoir quatre circuits fluidiques 9 parallèles dans l'exemple représenté. Les circuits fluidiques 9 sont par exemple formés dans l'épaisseur du support 6 et séparés par des cloisons 11. Les circuits fluidiques 9 peuvent ainsi former des canaux séparés au sein desquels la sueur peut circuler de manière indépendante. Le nombre de circuits fluidiques 9 peut être plus élevé ou plus faible que sur le dessin.

Chaque circuit fluidique 9 est muni d'un capteur 10A, 10B, 10C ou 10D. Les flèches 12 illustrent le sens d'écoulement de la sueur dans les circuits fluidiques 9. De préférence, les circuits fluidiques 9 aboutissent dans un réservoir de drainage qui retient les fluides analysés, de manière à éviter de remettre les produits de réaction de l'électrolyse en contact avec la peau du sujet.

Les capteurs 10A, 10B, 10C et 10D. agencés dans les circuits fluidiques 9 pour analyser la sueur sont de préférence des capteurs électrochimiques. Le principe de fonctionnement d'un capteur électrochimique est d'électrolyser la solution présente dans le circuit fluidique 9 entre une électrode de travail et une contre-électrode. Un tel capteur peut être réalisé de différentes manières, notamment de manière miniaturisée avec des dimensions de l'ordre du millimètre.

On va maintenant décrire plusieurs exemples de réalisation des capteurs électrochimiques en référence à la figure 6.

## Exemples

### Exemple 1

Le capteur 10A est destiné à détecter le peroxyde d'hydrogène. Il fonctionne donc avec une différence de potentiel égale au potentiel d'oxydation du peroxyde d'hydrogène $E_{H2O2}$. Le capteur 10B est destiné à détecter le monoxyde d'azote. Il fonctionne donc avec une différence de potentiel égale au potentiel d'oxydation du monoxyde d'azote $E_{NO}$. Le capteur 10C est destiné à détecter l'ion nitrite. Il fonctionne donc avec une différence de potentiel égale au potentiel d'oxydation de l'ion nitrite $E_{NO2^-}$.

Les capteurs 10A, 10B, 10C effectuent des mesures des intensités instantanées, notées $i_{oxdn}$, des courants Faradiques liés à l'oxydation électrochimique des espèces chimiques précitées. Les capteurs 10a, 10B, 10C permettent ainsi la détection et quantification de la concentration instantanée des espèces chimiques précitées.

Chacune des trois espèces chimiques précitées est détectable par des mesures ampérométriques à l'aide de microélectrodes. Celles-ci sont par exemple constituées de bandes de platine recouvertes d'une fine couche, par exemple micrométrique, de noir de platine déposé par réduction électrochimique en milieu aqueux de l'anion d'un sel de platine, $Pt(Cl)_6{}^{4-}$.

Les trois espèces chimiques ($NO$, $NO_2^-$ et $H_2O_2$) sont distinguables grâce au fait que leurs potentiels d'oxydation sur ces électrodes sont bien séparés, intervenant dans l'ordre suivant : $E_{H2O2} < E_{NO} < E_{NO2^-}$. Cependant les courants Faradiques sont additifs. Le courant mesuré au potentiel d'oxydation de chaque espèce chimique additionne donc des courants élémentaires liés à l'oxydation de cette espèce chimique et à l'oxydation de toutes les espèces chimiques dont les potentiels d'oxydation sont inférieurs.

Ainsi, seule l'espèce $H_2O_2$ est oxydable au potentiel d'oxydation $EH_2O_2$. Les espèces $H_2O_2$ et $NO$ sont oxydables au potentiel d'oxydation $E_{NO}$. Les trois espèces sont oxydables au potentiel d'oxydation $E_{NO2^-}$. Les courants mesurés par les capteurs 10A à 10C, notés respectivement $i_{oxdn}(EH_2O_2)$, $i_{oxdn}(E_{NO})$ et $i_{oxdn}(E_{NO2^-})$ obéissent donc aux équations suivantes :

$$i_{oxdn}(E_{H2O2}) = a1\ i_{H2O2}$$

$$i_{oxdn}(E_{NO}) = a2\ i_{H2O2} + a3\ i_{NO}$$

$$i_{oxdn}(E_{NO2^-}) = a4\ i_{H2O2} + a5\ i_{NO} + a6\ i_{NO2^-}$$

où les coefficients a1 à a6 représentent des constantes de calibration des capteurs, pouvant être mesurées expérimentalement.

On obtient ainsi par des soustractions facilement implémentées sur un circuit électronique :

$$i_{H2O2} = (1/a1)\ i_{oxdn}\ (E_{H2O2})$$

$$i_{NO} = (1/a3)\ i_{oxdn}\ (E_{NO}) - (a2/a1).(1/a3)\ i_{oxdn}\ (E_{H2O2})$$

$$i_{NO2^-} = (1/a6)\ i_{oxdn}\ (E_{NO2^-}) - (a4/a6)\ i_{H2O2} - (a5/a6)\ i_{NO}$$

A tout instant t, l'intensité instantanée du courant Faradique d'oxydation, , $i_S(t)$, de chaque espèce chimique S est proportionnelle à sa concentration, $C_S(t)$, dans le volume de fluide situé au-dessus des électrodes qui la détectent. Le facteur de proportionnalité dépend d'un facteur de forme, noté $\gamma$, qui est une fonction de la géométrie du capteur, et du nombre de Faraday, noté $n_S$, consommé par mole de l'espèce chimique, à savoir :

$$n_{H2O2} = n_{NO2^-} = 2\ \text{et}\ n_{NO} = 1$$

On rappelle que F désigne le Faraday, c'est-à-dire 96 500 Coulombs, la valeur de la charge d'une mole d'électrons.

Le facteur de forme $\gamma$ est un facteur constant imposé par la géométrie du dispositif électrochimique, évaluable théoriquement et mesurable expérimentalement par calibration. Par mesure de simplicité, on considère ci-dessous que les trois capteurs 10A à 10C ont des géométries identiques et de sorte que le facteur de forme $\gamma$ est commun à tous les capteurs.

Il en résulte que les concentrations des espèces chimiques peuvent être obtenues à partir des courants mesurés par les capteurs 10A à 10C, à l'aide des expressions suivantes, où la variable temporelle t a été explicitée :

$$C_{H2O2}(t) = i_{oxdn}(E_{H2O2}, t)/(2F\gamma)$$

$$C_{NO}(t) = [i_{oxdn}(E_{NO}, t) - i_{oxdn}(E_{H2O2}, t)]/(F\gamma)$$

$$C_{NO2^-}(t) = [i_{oxdn}(E_{NO2^-}, t) - i_{oxdn}(E_{NO}, t)]/(2F\gamma)$$

Dans l'exemple 1, les trois capteurs 10A à 10C peuvent donc fonctionner en parallèle, chacun avec un potentiel d'oxydation constant, à savoir $E_{H2O2}$, $E_{NO}$, et $E_{NO2^-}$ respectivement.

Selon une variante de réalisation, il est détecté uniquement le NO et le $NO_2^-$. Ce mode de réalisation est particulièrement avantageux lorsque la mesure de $H_2O_2$ n'est pas significative et n'influence pas les résultats de l'objectif visé. La concentration $C_{H2O2}(t)$ présentée ci-dessus est alors considérée comme uniformément nulle, soit $C_{H2O2}(t) = 0$. Le système d'équations est donc simplifié.

Exemple 2

Dans l'exemple 2, un seul circuit fluidique 9 et un seul capteur 10A sont utilisés, les autres pouvant être supprimés.

Dans ce cas, le capteur 10A fonctionne séquentiellement pour détecter les espèces chimiques précitées au cours de trois étapes successives. Le potentiel d'oxydation est donc commuté entre trois paliers de potentiel respectivement égaux aux trois potentiels d'oxydation précités, par exemple périodiquement selon la séquence $E_{H2O2} \rightarrow E_{NO} \rightarrow$

$$E_{NO2^-} \rightarrow E_{H2O2} \rightarrow E_{NO} \rightarrow E_{NO2^-} \rightarrow etc.$$

Dans ce cas, chaque potentiel d'oxydation est maintenu pendant une durée très grande par rapport à la constante de temps de l'électrode de travail, cette constante de temps étant par exemple quelques millisecondes pour des microélectrodes implémentées dans des canaux microfluidiques, et des mesures du courant sont faites à la fin de chaque palier de potentiel.

Pour le reste, le traitement des signaux de mesure peut être réalisé selon les mêmes équations qu'à l'exemple 1.

Exemple 3

Le monoxyde d'azote étant une petite molécule à la fois hydrophile et lipophile, elle peut aisément traverser de fines couches de polymère organique, au contraire des deux autres espèces $H_2O_2$ et $NO_2^-$. Elle peut donc être détectée isolément à l'aide d'un capteur électrochimique protégé par une telle couche filtrante, par exemple avec une électrode de travail en platine platiné revêtue une fine couche d'eugénol (4-allyl-2-méthoxyphénol) déposée par électropolymérisation.

Dans l'exemple 3, le capteur 10D est ainsi protégé par la couche filtrante représentée schématiquement au chiffre 19. La concentration instantanée de monoxyde d'azote peut ainsi être mesurée indépendamment de celle des espèces chimiques $H_2O_2$ et $NO_2^-$, selon l'expression :

$$C_{NO}(t) = [i_{oxdn}(E_{NO}, t)]_{eugénol}/(F\gamma) .$$

Où $i_{oxdn}(E_{NO}, t)]_{eugénol}$ désigne le courants mesuré par le capteur 10D.

Les autres capteurs 10A à 10C et les autres circuits fluidiques 9 peuvent être supprimés. Cette méthode peut donc être avantageusement utilisée avec un seul capteur lorsque seule la concentration de NO est recherchée.

En variante, le filtre 19 peut être agencé à d'autres positions entre l'élément collecteur 5 et le capteur 10D. La fonction du filtre 19 est de filtrer la sueur pour éviter que certains éléments contenus dans celle-ci ne viennent perturber la mesure du NO dissous dans la sueur. Ces éléments perturbateurs sont par exemple le peroxynitrite (ONOO-) ou le peroxyde d'hydrogène ($H_2O_2$).

Exemple 4

On cumule dans ce cas le capteur 10D de l'exemple 3 avec les capteurs 10A à 10C de l'exemple 1 ou avec le capteur 10A de l'exemple 2. Cette configuration permet d'obtenir deux mesures indépendantes de la concentration du monoxyde d'azote dissous et donc de contrôler la cohérence des mesures en vérifiant en particulier l'absence de dérive des capteurs, par exemple liée à une désactivation partielle de la surface de l'une des électrodes.

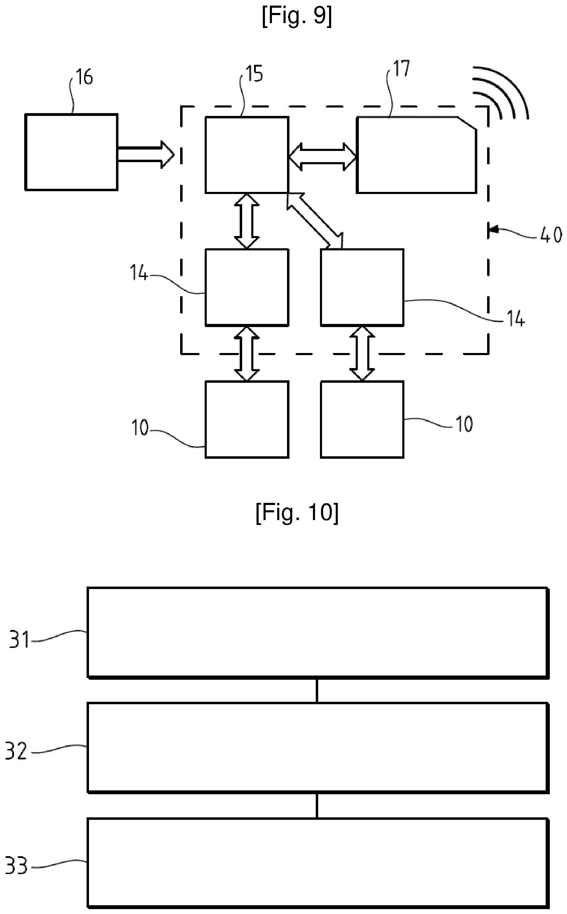

Dans ce cas, le dispositif électronique de contrôle électrochimique 40 (Fig. 9) est de préférence configuré pour comparer les deux mesures de la concentration du monoxyde d'azote et émettre une alarme lorsque le résultat de la comparaison remplit un critère prédéfini, par exemple dépasse un seuil prédéfini.

Dans les exemples 1 à 4 ci-dessus, les courant Faradiques instantanés mesurés permettent de mesurer la concentration des espèces chimiques dans la solution analysée. Par conséquent, dans un système statique, l'intensité du courant suffit à rendre compte de la production de l'espèce détectée.

Cependant, lorsque l'appareil de détection 1 est appliqué à un système physiologique essentiellement dynamique, il est souhaitable de pouvoir aussi accéder quantitativement à la dynamique de production de chaque espèce chimique par le système cardiovasculaire, par exemple lors de tests d'effort ou lors de suivis médicaux. Dans des conditions dynamiques, pour accéder à la quantité instantanée d'une espèce chimique, notée $\Delta Q(t)$, produite pendant une petite durée de temps, notée $\Delta t(t)$, il est souhaitable de connaître simultanément la concentration moyenne, $C_S(t)$, de l'espèce chimique et le débit volumique du fluide analysé, à savoir :

$$d(t) = (\Delta V/\Delta t)$$

où $\Delta V$ désigne le volume balayé pendant l'intervalle de temps $\Delta t$. Ainsi, l'intensité du flux de production, notée $P_S(t)$, d'une espèce chimique S à un instant t est donnée par :

$$P_S(t) = [\Delta Q/\Delta t](t) = C_S(t).d(t)$$

où la concentration moyenne $C_S(t)$ est obtenue à partir des intensités moyennes des courants d'oxydation électrochimique mesurés entre les instants t et t+$\Delta$t.

Dans le cadre des applications dynamiques envisagées, il est donc souhaitable que l'appareil de détection 1 mesure à la fois et à chaque instant t requis par la précision souhaitée pour le suivi temporel de l'état physiologique du patient, par exemple une fois par minute, les intensités moyennes, $i_{av}(t)$, du courant Faradique lié à l'oxydation électrochimique de la ou des espèces chimiques suivies et la valeur du débit volumique d(t) de sueur au temps t dans le circuit fluidique correspondant.

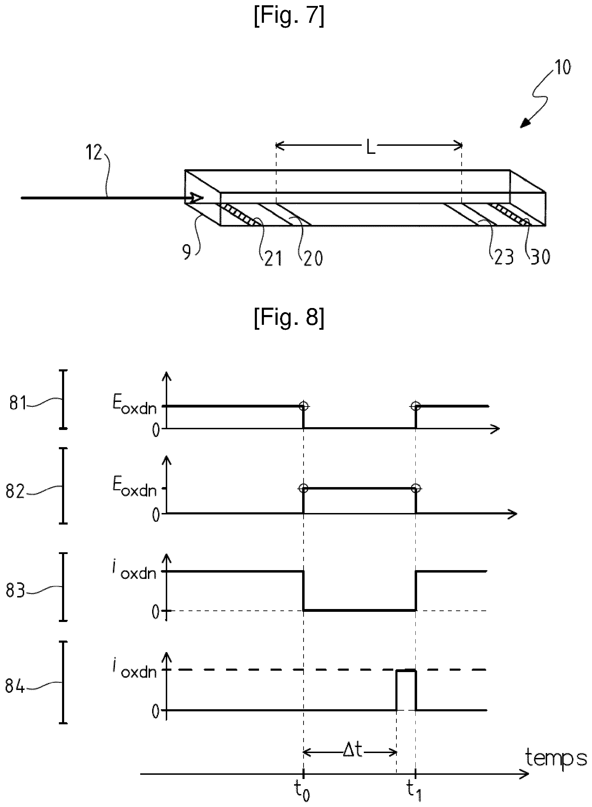

La figure 7 illustre un mode de réalisation d'un capteur électrochimique 10 pouvant répondre à ce double besoin de manière intégrée. Ce capteur électrochimique 10 comporte au moins une microélectrode bande formant une électrode de travail 20 ou une paire d'électrodes de travail 20, 23. Une telle microélectrode bande peut être réalisée en platine platiné (noir de platine), recouverte ou non d'une couche micrométrique d'eugénol électropolymérisée. Une telle microélectrode bande peut être implantée par microfabrication, par exemple CVD et/ou lithographie. Cette ou ces microélectrodes bandes permettent d'oxyder électrochimiquement les espèces chimiques choisies.

Le circuit fluidique 9 de la figure 7 est en outre équipé d'une électrode de référence 21, réalisée par exemple sous la forme d'une microbande d'Ag/AgCl, et placée en amont de l'électrode de travail 20 ou de la paire d'électrodes de travail 20, 23. Le circuit fluidique 9 est enfin équipé d'une contre-électrode 30 en platine platiné placée en aval de l'électrode de travail 20 ou de la paire d'électrodes de travail 20, 23. Nonobstant la représentation schématique fonctionnelle de la figure 7, la surface de la contre-électrode 30 est en réalité deux à trois fois plus grande que celle des autres électrodes.

L'ensemble du circuit fluidique 9 avec les électrodes 20, 21, 23, 30 est baigné par une lame de sueur non représentée, et constitue ainsi une cellule électrochimique microfluidique à trois ou quatre électrodes. Chacune des électrodes 20, 21, 23, 30 est connectée avec un dispositif électronique de contrôle électrochimique 40 (Fig. 9) grâce à des contacts électriques isolés de l'élément collecteur 5 et de la sueur.

Ce mode de réalisation d'un capteur électrochimique 10 peut être employé dans un ou plusieurs des circuits fluidiques 9 précités.

Pour mesurer le débit volumique d(t), le capteur électrochimique 10 doit comporter la paire d'électrodes de travail 20, 23. La solution décrite ici est simple et facilement industrialisable, car sans pièce mobile ni aucune hypothèse sur le régime hydrodynamique. Elle ne nécessite aucune intervention visant à moduler le débit du fluide, tout en étant adaptée à tout débit physiologique raisonnable.

Les deux électrodes de travail 20, 23, par exemple deux bandes de platine platiné pouvant servir de microélectrodes de travail, sont électriquement indépendantes et sont espacées d'une distance L le long de la trajectoire du fluide analysé dans le circuit fluidique 9. Les deux électrodes de travail 20 et 23 sont par exemple implantées sur le fond d'un canal linéaire dont la section présente une aire constante A.

L'électrode de travail 23 placée en aval est utilisée selon le procédé illustré sur la figure 8, qui comporte deux étapes. Le graphique 81 représente le potentiel électrique appliqué à l'électrode de travail 20 en fonction du temps. Le graphique 82 représente le potentiel électrique appliqué à l'électrode de travail 23 en fonction du temps. Les potentiels indiqués comme '0' sur les graphiques 81 et 82 signifient en réalité une déconnexion de l'électrode correspondante (circuit ouvert). Le graphique 83 représente le courant faradique mesuré à l'électrode de travail 20 en fonction du temps. Le graphique 84 représente le courant faradique mesuré à l'électrode de travail 23 en fonction du temps.

Pendant une première étape effectuée sur une plage de temps antérieure à l'instant ta, le potentiel $E_{oxdn}$ appliqué à l'électrode de travail 20 est adéquat pour permettre l'oxydation de la ou des espèces chimiques visées, tandis que l'électrode de travail aval 23 est déconnectée. L'électrode de travail 20 placée en amont permet alors d'enregistrer en continu le courant électrochimique instantané, $i_{oxdn}(t)$, indique ainsi, à la suite des éventuels calculs indiqués plus haut, la concentration $C(t)$ de la ou des espèces chimiques visées dans le fluide analysé.

Pendant une deuxième étape effectuée sur une plage de temps à partir de l'instant t0, l'électrode de travail 20 est déconnectée et le potentiel $E_{oxdn}$ est appliqué à l'électrode de travail 23 aval.

A l'instant $t_0$, le flux de sueur passant au-dessus de l'électrode de travail 23 a déjà été électrolysé (complètement ou en partie) lors de son passage au-dessus de l'électrode de travail 20 qui est située en amont, de sorte que la concentration de l'espèce chimique visée y est nulle ou tout au moins bien plus faible qu'avant son entrée dans le dispositif électrochimique. L'intensité $i_{oxdn}$ du courant détecté par l'électrode de travail 23 (graphique 84) est donc nulle (ou tout au moins bien plus faible que celle du courant $i_{oxdn}$ détecté à l'électrode de travail 20 avant l'instant $t_0$.

A l'instant $t_0 + \Delta t$ l'électrode de travail 23 commence à analyser une solution non électrolysée et l'intensité du courant $i_{oxdn}$ qu'elle détecte devient du même ordre que celle détectée par l'électrode de travail 20 avant l'instant $t_0$. La croissance du courant, schématisé par un échelon sur la figure 8, est détectée par un circuit électronique ad-hoc. La durée $\Delta t$, qui est le retard entre cette croissance et le moment t0 de la déconnection de l'électrode de travail 20, représente le temps nécessaire au flux de sueur pour transiter entre les deux électrodes de travail 20 et 23. La durée $\Delta t$ est représentée par une double flèche en bas de la Figure 8. Afin de simplifier la représentation, on a supposé sur la figure 8 que l'électrolyse de l'espèce chimique visée est complète lorsque l'électrode de travail 20 est connectée. Les mêmes principes de mesure sont applicables lorsque cette électrolyse est seulement partielle.

La vitesse de l'écoulement v(t) et le débit d(t) peuvent donc être estimé comme suit :

$$v(t) = L/\Delta t$$

$$d(t) = A.v(t)$$

Le potentiel $E_{oxdn}$ appliqué à l'électrode de travail 23 est adéquat pour permettre l'oxydation de la ou des espèces chimiques visées, tandis que l'électrode de travail 20 est déconnectée. La mesure de concentration peut donc éventuellement se poursuivre pendant une certaine durée avec l'électrode de travail 23. La deuxième étape se termine par la déconnexion de l'électrode de travail 23 à l'instant $t_1$. L'électrode de travail 20 peut être alors reconnectée et le procédé peut être réitéré autant que de nécessaire pour évaluer le débit d(t) à des instants successifs.

La distance L entre les deux électrodes de travail 20 et 23 est de préférence suffisamment petite, par exemple de l'ordre de 1 mm, pour que les changements de la réponse physiologique du patient soient négligeables pendant la durée $\Delta t$.

Le procédé de mesure de débit décrit ci-dessus peut être employé simultanément dans tous les circuits fluidiques parallèles. Toutefois, si ces circuits sont configurés et alimentés de manière similaire, une seule mesure de débit peut être suffisante. Dans ce cas, le procédé de mesure de débit décrit ci-dessus peut être employé dans un seul circuit fluidique 9. Par ailleurs, ce procédé de mesure de débit est combinable avec les capteurs des différents exemples 1 à 4 décrits plus haut.

Les procédés de détection de concentration et de débit décrits ci-dessus peuvent être mis en oeuvre de manière automatisée à l'aide d'un dispositif électronique de contrôle 40, qui est de préférence intégré à l'appareil de détection 1

En référence à la figure 9, on décrit maintenant un mode de réalisation du dispositif électronique de contrôle 40 pouvant être intégré dans l'appareil de détection 1, par exemple sous la forme d'une carte électronique 13 représentée sur la figure 3.

Le ou chaque capteur électrochimique 10 est connecté à un convertisseur analogique numérique 14, qui alimente lui-même un processeur 15. Le processeur 15 est par exemple programmé pour mettre en oeuvre les procédés de détection de concentration et de débit décrits ci-dessus.

Une source d'énergie 16, par exemple une batterie, alimente le dispositif électronique de contrôle 40. Un module de communication 17, filaire ou non filaire, peut aussi être prévu pour communiquer les résultats des mesures de concentration, de débit et/ou ou de flux de quantité de matière, pour une ou chaque espèce chimique visée, à un appareil de stockage ou de post-traitement.

La figure 10 représente un procédé pouvant être mis en oeuvre par le processeur 15 dans un mode de réalisation.

A l'étape 31, la concentration instantanée Cs(t) d'une espèce chimique S est déterminée à partir des mesures électrochimiques.

A l'étape 32, le débit volumique d(t) dans le circuit fluidique correspondant est déterminé.

A l'étape 33, le flux de quantité de matière pour l'espace chimique considérée est déterminé sur la base de Cs(t) et d(t), par exemple :

$$P_S(t) = C_S(t).d(t)$$

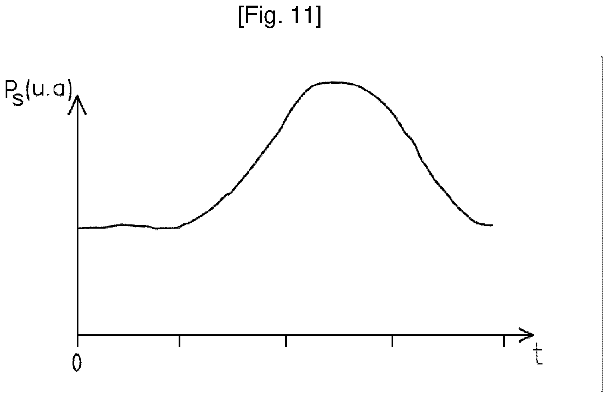

La figure 11 est un graphique illustrant un signal de mesure de flux de quantité de matière en fonction du temps pouvant être obtenu avec l'appareil de détection 1, par exemple au cours d'un test d'effort d'un sujet pour l'espèce NO.

**[0108]** Le dispositif électronique de contrôle 40 comporte éventuellement d'autres modules fonctionnels, par exemple un module gyroscopique et/ou accélérométrique pour détecter l'orientation et les mouvements du sujet ainsi que le niveau d'activité du sujet, et un capteur de température pour mesurer la température de l'épiderme du sujet. Il est utile de connaître la température de la peau en raison des corrélations entre la température et la dilatation des vaisseaux.

Certains éléments de l'appareil de détection 1, notamment le dispositif électronique de contrôle 40 peuvent être réalisés sous différentes formes, de manière unitaire ou distribuée, au moyen de composants matériels et/ou logiciels. Des composants matériels utilisables sont les circuits intégrés spécifiques ASIC, les réseaux logiques programmables FPGA ou les microprocesseurs. Des composants logiciels peuvent être écrits dans différents langages de programmation, par exemple C, C++, Java ou VHDL. Cette liste n'est pas exhaustive.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre des revendications.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Appareil de détection (1) pour détecter au moins une espèce chimique dissoute dans la sueur d'un sujet humain ou animal, ladite au moins une espèce chimique dissoute incluant le monoxyde d'azote NO, ledit appareil comportant :

   un élément collecteur (5) destiné à être posé sur une zone d'investigation d'un épiderme (2) dudit sujet, et
   un dispositif détecteur comportant au moins un circuit fluidique (8, 9) couplé à l'élément collecteur pour conduire au moins un flux de sueur provenant de la zone d'investigation et un capteur électrochimique (10) comportant des électrodes disposées dans le circuit fluidique, le capteur électrochimique étant configuré pour produire au moins un signal représentatif d'une concentration du monoxyde d'azote NO dissout dans le flux de sueur et un signal représentatif d'une vitesse d'écoulement du flux de sueur.

2. Appareil selon la revendication 1, dans lequel le dispositif détecteur est configuré pour produire un signal représentatif d'une production instantanée du monoxyde d'azote NO dans la zone d'investigation sur la base du signal représentatif de la concentration du monoxyde d'azote NO et du signal représentatif de la vitesse d'écoulement du flux de sueur.

3. Appareil selon la revendication 1 ou 2, dans lequel le capteur électrochimique (10) comporte au moins une électrode de travail (20) et une contre-électrode (30) disposées dans le circuit fluidique, le capteur électrochimique étant configuré pour produire le signal représentatif de la concentration du monoxyde d'azote NO par une mesure électrique, notamment ampérométrique, entre la au moins une électrode de travail et la contre-électrode.

**4.** Appareil selon la revendication 3, dans lequel le capteur électrochimique comporte une électrode de travail amont (20) et une électrode de travail aval (23) espacées dans le circuit fluidique dans une direction d'écoulement (12) du flux de sueur, le capteur électrochimique étant configuré pour produire le signal représentatif de la vitesse d'écoulement par mesure d'un retard ($\Delta t$) entre une variation de courant dans l'électrode de travail amont et une variation de courant dans l'électrode de travail aval.

**5.** Appareil selon l'une des revendications 1 à 4, dans lequel le capteur électrochimique (10D) est configuré pour polariser les électrodes à un potentiel électrique d'oxydation du monoxyde d'azote NO et dans lequel le dispositif détecteur comporte un filtre (19) configuré pour filtrer le flux de sueur atteignant le capteur électrochimique afin d'éliminer le peroxyde d'hydrogène.

**6.** Appareil selon l'une des revendications 1 à 4, dans lequel ladite au moins une espèce chimique inclut en outre l'ion nitrite $NO_2^-$ et/ou le peroxyde d'hydrogène $H_2O_2$.

**7.** Appareil selon la revendication 6, dans lequel le capteur électrochimique (10A) est configuré pour détecter séquentiellement plusieurs espèces chimiques au cours d'une pluralité d'étapes de mesure, le capteur électrochimique étant configuré pour polariser les électrodes à un potentiel électrique d'oxydation du peroxyde d'hydrogène $H_2O_2$ au cours d'une première étape et polariser les électrodes à un potentiel électrique d'oxydation du monoxyde d'azote NO au cours d'une deuxième étape, et dans lequel le dispositif détecteur est configuré pour produire le signal représentatif d'une concentration de monoxyde d'azote NO sur la base d'un premier signal de mesure ampérométrique obtenu lors de la première étape et d'un deuxième signal de mesure ampérométrique obtenu lors de la deuxième étape.

**8.** Appareil selon la revendication 7, dans lequel le capteur électrochimique (10A) est configuré pour polariser les électrodes à un potentiel électrique d'oxydation de l'ion nitrite $NO_2^-$ au cours d'une troisième étape, et dans lequel le dispositif détecteur est configuré pour produire un signal représentatif d'une concentration de l'ion nitrite $NO_2^-$ sur la base desdits premier et deuxième signaux de mesure ampérométriques et d'un troisième signal de mesure ampérométrique obtenu lors de la troisième étape.

**9.** Appareil selon la revendication 6, dans lequel le dispositif détecteur comporte :

un premier circuit fluidique (9) couplé à l'élément collecteur pour conduire un premier flux de sueur provenant de la zone d'investigation et un premier capteur électrochimique (10A) comportant des électrodes disposées dans le premier circuit fluidique, le premier capteur électrochimique étant configuré pour polariser les électrodes à un potentiel électrique d'oxydation du peroxyde d'hydrogène $H_2O_2$, et un deuxième circuit fluidique (9) couplé à l'élément collecteur pour conduire un deuxième flux de sueur provenant de la zone d'investigation et un deuxième capteur électrochimique (10B) comportant des électrodes disposées dans le deuxième circuit fluidique, le deuxième capteur électrochimique étant configuré pour polariser les électrodes à un potentiel électrique d'oxydation du monoxyde d'azote NO,

dans lequel le dispositif détecteur est configuré pour produire un signal représentatif d'une concentration de monoxyde d'azote NO sur la base d'un premier signal de mesure ampérométrique produit par le premier capteur électrochimique (10A) et d'un deuxième signal de mesure ampérométrique produit par le deuxième capteur électrochimique (10B).

**10.** Appareil selon la revendication 9, dans lequel le dispositif détecteur (5, 6) comporte en outre :
un troisième circuit fluidique (9) couplé à l'élément collecteur pour conduire un troisième flux de sueur provenant de la zone d'investigation et un troisième capteur électrochimique (10C) comportant des électrodes disposées dans le troisième circuit fluidique, le troisième capteur électrochimique étant configuré pour polariser les électrodes à un potentiel électrique d'oxydation de l'ion nitrite $NO_2^-$, et dans lequel le dispositif détecteur est configuré pour produire un signal représentatif d'une concentration de l'ion nitrite $NO_2^-$ sur la base desdits premier et deuxième signaux de mesure ampérométriques et d'un troisième signal de mesure ampérométrique produit par le troisième capteur électrochimique.

**11.** Appareil selon la revendication 9 ou 10, dans lequel le dispositif détecteur comporte en outre :
un quatrième circuit fluidique (9) couplé à l'élément collecteur pour conduire un quatrième flux de sueur provenant de la zone d'investigation et un quatrième capteur électrochimique (10D) comportant des électrodes disposées dans le quatrième circuit fluidique, dans lequel le quatrième capteur électrochimique est configuré pour polariser les électrodes à un potentiel électrique d'oxydation du monoxyde d'azote NO et dans lequel le quatrième circuit fluidique comporte un filtre (19) configuré pour filtrer le flux de sueur atteignant le quatrième capteur électrochimique afin d'éliminer le peroxyde d'hydrogène H$_2$O$_2$.

12. Appareil selon la revendication 5 ou 11, dans lequel le filtre (19) comporte une couche d'eugénol (4-allyl-2méthoxyphénol) déposée sur au moins une électrode du capteur électrochimique (10D).

13. Appareil selon la revendication 12, dans lequel le dispositif détecteur est configuré pour comparer une mesure de la concentration du monoxyde d'azote obtenue avec le quatrième capteur électrochimique (10D) avec une mesure de la concentration du monoxyde d'azote obtenue avec les premier et deuxième capteurs électrochimiques (10A, 10B) et émettre une alarme lorsque le résultat de la comparaison remplit un critère prédéfini.

14. Appareil selon l'une des revendications 1 à 13, dans lequel l'élément collecteur (5) comporte un corps fibreux pour conduire la sueur par capillarité.

15. Appareil selon l'une des revendications 1 à 14, comportant en outre une enveloppe (7) entourant l'élément collecteur (5) de manière à former une barrière étanche autour de la zone d'investigation par contact avec l'épiderme (2) dudit sujet.

16. Appareil selon l'une des revendications 1 à 15, comportant en outre un dispositif de communication (17) configuré pour transmettre un ou plusieurs signaux de mesure produits par le dispositif détecteur à destination d'un appareil de stockage ou de post-traitement.

17. Appareil selon l'une des revendications 1 à 16, dans lequel le circuit fluidique (9) est formé dans un support isolant (6) et dans lequel les électrodes du capteur électrochimique (10) sont constituées de dépôts métalliques sur ledit support isolant, les dépôts métalliques étant sélectionnés dans le groupe consistant en l'argent, l'or, le platine et le noir de platine

18. Appareil selon l'une des revendications 1 à 17, comportant en outre un module gyroscopique et/ou au moins un accéléromètre pour détecter un état d'activité du sujet.

19. Appareil selon l'une des revendications 1 à 18, comportant en outre un capteur de température pour mesurer la température de l'épiderme du sujet.

20. Procédé pour déterminer la production (Ps) d'au moins une espèce chimique par un sujet humain ou animal, ladite au moins une espèce chimique incluant le monoxyde d'azote NO, le procédé comportant :

choisir une zone d'investigation d'un épiderme dudit sujet,
appliquer un appareil (1) selon l'une des revendications 1 à 19 pendant une durée nécessaire pour produire le signal représentatif d'une concentration du monoxyde d'azote NO dissout dans le flux de sueur et le signal représentatif d'une vitesse d'écoulement du flux de sueur, et
déterminer une mesure de la production (Ps) du monoxyde d'azote NO par le sujet à partir du signal représentatif de la concentration du monoxyde d'azote NO dissout dans le flux de sueur et du signal représentatif de la vitesse d'écoulement du flux de sueur.

21. Procédé selon la revendication 20, comportant en outre :
évaluer une souffrance des tissus vasculaires du sujet à partir de la mesure de la production du monoxyde d'azote NO.

22. Procédé selon la revendication 20, comportant en outre :
évaluer une capacité cardiovasculaire du sujet à partir de la mesure de la production du monoxyde d'azote NO. ;

**Patentansprüche**

1. Detektionsvorrichtung (1) zum Detektieren mindestens einer chemischen Spezies, die im Schweiß eines menschlichen oder tierischen Probanden gelöst ist, wobei die mindestens eine gelöste chemische Spezies Stickstoffmonoxid NO umfasst, wobei die Vorrichtung umfasst:

ein Sammelelement (5), das dazu bestimmt ist, auf einem Untersuchungsbereich einer Epidermis (2) des Probanden angeordnet zu werden, und

eine Detektorvorrichtung umfassend mindestens einen Fluidkreislauf (8, 9), der mit dem Sammelelement verbunden ist, um mindestens einen Schweißstrom aus dem Untersuchungsbereich zu leiten, und einen elektrochemischen Sensor (10) umfassend Elektroden, die in dem Fluidkreislauf angeordnet sind, wobei der elektrochemische Sensor so konfiguriert ist, dass dieser mindestens ein Signal, das eine Konzentration von in dem Schweißstrom gelöstem Stickstoffmonoxid NO darstellt, und ein Signal, das eine Fließgeschwindigkeit des Schweißstroms darstellt, erzeugt.

2. Vorrichtung nach Anspruch 1, wobei die Detektorvorrichtung so konfiguriert ist, dass diese auf der Grundlage des Signals, das die Konzentration von Stickstoffmonoxid NO darstellt, und des Signals, das die Fließgeschwindigkeit des Schweißstroms darstellt, ein Signal erzeugt, das eine instantane Produktion von Stickstoffmonoxid NO im Untersuchungsbereich darstellt.

3. Vorrichtung nach Anspruch 1 oder 2, wobei der elektrochemische Sensor (10) mindestens eine Arbeitselektrode (20) und eine Gegenelektrode (30) umfasst, die im Fluidkreislauf angeordnet sind, wobei der elektrochemische Sensor so konfiguriert ist, dass dieser das Signal, das die Konzentration von Stickstoffmonoxid NO darstellt, durch eine elektrische, insbesondere amperometrische Messung, zwischen der mindestens einen Arbeitselektrode und der Gegenelektrode erzeugt.

4. Vorrichtung nach Anspruch 3, wobei der elektrochemische Sensor eine stromaufwärtige Arbeitselektrode (20) und eine stromabwärtige Arbeitselektrode (23) umfasst, die in dem Fluidkreislauf in einer Fließrichtung (12) des Schweißstroms beabstandet sind, wobei der elektrochemische Sensor so konfiguriert ist, dass dieser das die Fließgeschwindigkeit darstellende Signal durch Messen einer Verzögerung ($\Delta t$) zwischen einer Stromänderung in der stromaufwärtigen Arbeitselektrode und einer Stromänderung in der stromabwärtigen Arbeitselektrode erzeugt.

5. Vorrichtung nach einem der Ansprüche 1 bis 4, wobei der elektrochemische Sensor (10D) so konfiguriert ist, dass dieser die Elektroden auf ein elektrisches Potential zur Oxidation von Stickstoffmonoxid NO polarisiert, und wobei die Detektorvorrichtung einen Filter (19) umfasst, der so konfiguriert ist, dass dieser den den elektrochemischen Sensor erreichenden Schweißstrom filtert, um Wasserstoffperoxid zu entfernen.

6. Vorrichtung nach einem der Ansprüche 1 bis 4, wobei die mindestens eine chemische Spezies zusätzlich das Nitrit-Ion $NO_2^-$ und/oder das Wasserstoffperoxid $H_2O_2$ umfasst.

7. Vorrichtung nach Anspruch 6, wobei der elektrochemische Sensor (10A) so konfiguriert ist, dass dieser nacheinander mehrere chemische Spezies in einer Vielzahl von Messschritten detektiert, wobei der elektrochemische Sensor so konfiguriert ist, dass dieser die Elektroden in einem ersten Schritt auf ein elektrisches Potential zur Oxidation von Wasserstoffperoxid $H_2O_2$ polarisiert und in einem zweiten Schritt auf ein elektrisches Potential zur Oxidation von Stickstoffmonoxid NO polarisiert, und wobei die Detektorvorrichtung so konfiguriert ist, dass diese das Signal, das eine Konzentration von Stickstoffmonoxid NO darstellt, auf der Grundlage eines ersten amperometrischen Messsignals, das im ersten Schritt erhalten wurde, und eines zweiten amperometrischen Messsignals, das im zweiten Schritt erhalten wurde, erzeugt.

8. Vorrichtung nach Anspruch 7, wobei der elektrochemische Sensor (10A) so konfiguriert ist, dass dieser die Elektroden in einem dritten Schritt auf ein elektrisches Potential zur Oxidation des Nitrit-Ions $NO_2^-$ polarisiert, und wobei die Detektorvorrichtung so konfiguriert ist, dass diese auf der Grundlage des ersten und des zweiten amperometrischen Messsignals und eines in dem dritten Schritt erhaltenen dritten amperometrischen Messsignals ein Signal erzeugt, das eine Konzentration des Nitrit-Ions $NO_2^-$ darstellt.

9. Vorrichtung nach Anspruch 6, wobei die Detektorvorrichtung umfasst:

einen ersten Fluidkreislauf (9), der mit dem Sammelelement verbunden ist, um einen ersten Strom von Schweiß aus dem Untersuchungsbereich zu leiten, und einen ersten elektrochemischen Sensor (10A) mit Elektroden, die in dem ersten Fluidkreislauf angeordnet sind, wobei der erste elektrochemische Sensor so konfiguriert ist, dass dieser die Elektroden auf ein elektrisches Potential zur Oxidation von Wasserstoffperoxid $H_2O_2$ polarisiert, und einen zweiter Fluidkreislauf (9), der mit dem Sammelelement verbunden ist, um einen zweiten Strom von Schweiß aus dem Untersuchungsbereich zu leiten, und einen zweiten elektrochemischen Sensor (10B) mit Elektroden, die in dem zweiten Fluidkreislauf angeordnet sind, wobei der zweite elektrochemische Sensor so konfiguriert ist, dass dieser die Elektroden auf ein elektrisches Potential zur Oxidation von Stickstoffmonoxid NO polarisiert,

wobei die Detektorvorrichtung konfiguriert ist, um ein Signal zu erzeugen, das eine Konzentration von Stickstoffmonoxid NO auf der Grundlage eines ersten amperometrischen Messsignals, das von dem ersten elektrochemischen Sensor (10A) erzeugt ist, und eines zweiten amperometrischen Messsignals, das von dem zweiten elektrochemischen Sensor (10B) erzeugt ist, darstellt.

10. Vorrichtung nach Anspruch 9, wobei die Detektorvorrichtung (5, 6) weiterhin umfasst:

einen dritten Fluidkreislauf (9), der mit dem Sammelelement verbundet ist, um einen dritten Strom von Schweiß aus dem Untersuchungsbereich zu leiten, und einen dritten elektrochemischen Sensor (10C) mit Elektroden, die in dem dritten Fluidkreislauf angeordnet sind, wobei der dritte elektrochemische Sensor so konfiguriert ist, dass dieser die Elektroden auf ein elektrisches Potential zur Oxidation des Nitrit-Ions $NO_2^-$ polarisiert, und wobei die Detektorvorrichtung so konfiguriert ist, dass diese ein Signal erzeugt, das eine Konzentration des Nitrit-Ions $NO_2^-$ auf der Grundlage des ersten und des zweiten amperometrischen Messsignals und eines dritten amperometrischen Messsignals, das von dem dritten elektrochemischen Sensor erzeugt ist, darstellt.

11. Vorrichtung nach Anspruch 9 oder 10, wobei die Detektorvorrichtung ferner umfasst:

einen vierten Fluidkreislauf (9), der mit dem Sammelelement verbundet ist, um einen vierten Strom von Schweiß aus dem Untersuchungsbereich zu leiten, und einen vierten elektrochemischen Sensor (10D) mit Elektroden, die in dem vierten Fluidkreislauf angeordnet sind, wobei der vierte elektrochemische Sensor so konfiguriert ist, dass dieser die Elektroden auf ein elektrisches Potential zur Oxidation von Stickstoffmonoxid NO polarisiert, und wobei der vierte Fluidkreislauf einen Filter (19) umfasst, der so konfiguriert ist, dass er den den vierten elektrochemischen Sensor erreichenden Schweißstrom filtert, um Wasserstoffperoxid $H_2O_2$ zu entfernen.

12. Vorrichtung nach Anspruch 5 oder 11, wobei der Filter (19) eine Schicht aus Eugenol (4-Allyl-2methoxyphenol) umfasst, die auf mindestens einer Elektrode des elektrochemischen Sensors (10D) angeordnet ist.

13. Vorrichtung nach Anspruch 12, wobei die Detektorvorrichtung so konfiguriert ist, dass diese eine mit dem vierten elektrochemischen Sensor (10D) erhaltene Messung der Konzentration des Stickstoffmonoxids mit einer mit dem ersten und zweiten elektrochemischen Sensor (10A, 10B) erhaltenen Messung der Konzentration des Stickstoffmonoxids vergleicht und einen Alarm ausgibt, wenn das Ergebnis des Vergleichs ein vordefiniertes Kriterium erfüllt.

14. Vorrichtung nach einem der Ansprüche 1 bis 13, wobei das Sammelelement (5) einen faserigen Körper umfasst, um den Schweiß durch Kapillarwirkung zu leiten.

15. Vorrichtung nach einem der Ansprüche 1 bis 14 umfassend weiterhin eine Umhüllung (7), die das Sammelelement (5) umgibt, um durch Kontakt mit der Epidermis (2) des Probanden eine wasserdichte Barriere um den Untersuchungsbereich zu bilden.

16. Vorrichtung nach einem der Ansprüche 1 bis 15 umfassend weiterhin eine Kommunikationsvorrichtung (17) umfasst, die so konfiguriert ist, dass diese ein oder mehrere von der Detektorvorrichtung erzeugte Messsignale an eine Speicher- oder Nachverarbeitungsvorrichtung überträgt.

17. Vorrichtung nach einem der Ansprüche 1 bis 16, wobei der Fluidkreislauf (9) in einem isolierenden Träger (6) ausgebildet ist und wobei die Elektroden des elektrochemischen Sensors (10) aus Metallablagerungen auf dem isolierenden Träger bestehen, wobei die Metallabscheidungen aus der Gruppe ausgewählt sind, die aus Silber, Gold, Platin und Platinschwarz besteht.

18. Vorrichtung nach einem der Ansprüche 1 bis 17 umfassen weiterhin ein Kreiselmodul und/oder mindestens einen Beschleunigungsmesser zum Detektieren eines Aktivitätszustands des Probanden.

19. Vorrichtung nach einem der Ansprüche 1 bis 18 umfassen weiterhin einen Temperatursensor zum Messen der Temperatur der Epidermis des Probanden.

20. Verfahren zur Bestimmung der Erzeugung ($P_s$) von mindestens einer chemischen Spezies durch ein menschliches oder tierisches Probanden, wobei die mindestens eine chemische Spezies Stickstoffmonoxid NO umfasst, wobei das Verfahren umfasst:

Auswählen eines Untersuchungsbereichs einer Epidermis des Probanden,

Anlegen einer Vorrichtung (1) nach einem der Ansprüche 1 bis 19 für eine Zeitdauer, die erforderlich ist, um das Signal, das für eine Konzentration von in dem Schweißstrom gelöstem Stickstoffmonoxid NO dargestellt ist, und das Signal, das für eine Fließgeschwindigkeit des Schweißstroms dargestellt ist, zu erzeugen, und Bestimmen einer Messung der Erzeugung ($P_s$) von Stickstoffmonoxid NO durch die Probanden aus dem Signal, das für die Konzentration des im Schweißstrom gelösten Stickstoffmonoxids NO dargestellt ist, und aus dem Signal, das für die Fließgeschwindigkeit des Schweißstroms dargestellt ist.

21. Verfahren nach Anspruch 20 weiterhin umfassend:
Bewerten eines Leidens des vaskulären Gewebes des Probanden anhand der Messung der Herstellung von Stickstoffmonoxid NO.

22. Verfahren nach Anspruch 20, weiterhin umfassend:
Bewerten einer Herz-Kreislauf-Fähigkeit des Probanden anhand der Messung der Herstellung von Stickstoffmonoxid NO.

**Claims**

1. A detection apparatus (1) for detecting at least one chemical species dissolved in the sweat of a human or animal subject, said at least one dissolved chemical species including nitric oxide NO, said apparatus comprising:

   a collecting element (5) intended to be placed on an investigation zone of a subject's epidermis (2), and
   a detection device comprising at least one fluidic circuit (8, 9) coupled to the collecting element in order to conduct at least one flow of sweat originating from the investigation zone, and at least one electrochemical sensor (10) comprising electrodes disposed in the fluidic circuit, the electrochemical sensor being configured to produce at least a signal that is representative of a concentration of nitric oxide NO in the flow of sweat and a signal that is representative of a flow velocity of the flow of sweat.

2. The apparatus as claimed in claim 1, in which the detection device is configured to produce a signal that is representative of an instantaneous production of nitric oxide NO in the investigation zone on the basis of a signal that is representative of the concentration of nitric oxide NO and of a signal that is representative of the flow velocity of the flow of sweat.

3. The apparatus as claimed in claim 1 or claim 2, in which the electrochemical sensor (10) comprises at least one working electrode (20) and a counter-electrode (30) disposed in the fluidic circuit, the electrochemical sensor being configured to produce the signal that is representative of the concentration of the nitric oxide NO by an electrical measurement, in particular amperometric, between the at least one working electrode and the counter-electrode.

4. The apparatus as claimed in claim 3, in which the electrochemical sensor comprises an upstream working electrode (20) and a downstream working electrode (23) spaced apart in the fluidic circuit in a direction of flow (12) of the flow of sweat, the electrochemical sensor being configured to produce the signal that is representative of the flow velocity by measuring a delay ($\Delta t$) between a variation in current in the upstream working electrode and a variation in current in the downstream working electrode.

5. The apparatus as claimed in one of claims 1 to 4, in which the electrochemical sensor (10D) is configured to polarize the electrodes to an electrical potential for the oxidation of nitric oxide NO and in which the detection device comprises a filter (19) that is configured to filter the flow of sweat reaching the electrochemical sensor in order to eliminate hydrogen peroxide.

6. The apparatus as claimed in one of claims 1 to 4, in which said at least one chemical species additionally includes the nitrite ion $NO_2^-$ and/or hydrogen peroxide $H_2O_2$.

7. The apparatus as claimed in claim 6, in which the electrochemical sensor (10A) is configured for the sequential detection of several chemical species during a plurality of measurement steps, the electrochemical sensor being configured to polarize the electrodes to an electrical potential for the oxidation of hydrogen peroxide $H_2O_2$ during a first step and to polarize the electrodes to an electrical potential for the oxidation of nitric oxide NO during a second step, and in which the detection device is configured to produce the signal that is representative of a concentration of nitric oxide NO on the basis of a first amperometric measurement signal obtained during the first step and of a second amperometric measurement signal obtained during the second step.

8. The apparatus as claimed in claim 7, in which the electrochemical sensor (10A) is configured to polarize the electrodes to an electrical potential for the oxidation of the nitrite ion $NO_2^-$ during a third step, and in which the detection device is configured to produce a signal that is representative of a concentration of the nitrite ion $NO_2^-$ on the basis of said first and second amperometric measurement signals and of a third amperometric measurement signal obtained during the third step.

9. The apparatus as claimed in claim 6, in which the detection device comprises:

   a first fluidic circuit (9) coupled to the collecting element in order to conduct a first flow of sweat originating from the investigation zone and a first electrochemical sensor (10A) comprising electrodes disposed in the first fluidic circuit, the first electrochemical sensor being configured to polarize the electrodes to an electrical potential for the oxidation of hydrogen peroxide $H_2O_2$, and
   a second fluidic circuit (9) coupled to the collecting element in order to conduct a second flow of sweat originating from the investigation zone and a second electrochemical sensor (10B) comprising electrodes disposed in the second fluidic circuit, the second electrochemical sensor being configured to polarize the electrodes to an electrical potential for the oxidation of nitric oxide NO,
   in which the detection device is configured to produce a signal that is representative of a concentration of nitric oxide NO on the basis of a first amperometric measurement signal produced by the first electrochemical sensor (10A) and of a second amperometric measurement signal produced by the second electrochemical sensor (10B).

10. The apparatus as claimed in claim 9, in which the detection device (5, 6) additionally comprises:
    a third fluidic circuit (9) coupled to the collecting element in order to conduct a third flow of sweat originating from the investigation zone and a third electrochemical sensor (10C) comprising electrodes disposed in the third fluidic circuit, the third electrochemical sensor being configured to polarize the electrodes to an electrical potential for the oxidation of the nitrite ion $NO_2^-$, and in which the detection device is configured to produce a signal that is representative of a concentration of the nitrite ion $NO_2^-$ on the basis of said first and second amperometric measurement signals and of a third amperometric measurement signal produced by the third electrochemical sensor.

11. The apparatus as claimed in claim 9 or claim 10, in which the detection device additionally comprises:
    a fourth fluidic circuit (9) coupled to the collecting element in order to conduct a fourth flow of sweat originating from the investigation zone, and a fourth electrochemical sensor (10D) comprising electrodes disposed in the fourth fluidic circuit, in which the fourth electrochemical sensor is configured to polarize the electrodes to an electrical potential for the oxidation of nitric oxide NO and in which the fourth fluidic circuit comprises a filter (19) that is configured to filter the flow of sweat reaching the fourth electrochemical sensor in order to eliminate hydrogen peroxide $H_2O_2$.

12. The apparatus as claimed in claim 5 or claim 11, in which the filter (19) comprises a layer of eugenol (4-allyl-2methoxyphenol) deposited on at least one electrode of the electrochemical sensor (10D).

13. The apparatus as claimed in claim 12, in which the detection device is configured to compare a measurement of the concentration of nitric oxide obtained with the fourth electrochemical sensor (10D) with a measurement of the concentration of nitric oxide obtained with the first and second electrochemical sensors (10A, 10B) and to issue an alarm when the result of the comparison satisfies a predefined criterion.

14. The apparatus as claimed in one of claims 1 to 13, in which the collecting element (5) comprises a fibrous body for conducting the sweat by capillary action.

15. The apparatus as claimed in one of claims 1 to 14, additionally comprising an envelope (7) surrounding the collecting element (5) in a manner such as to form an impervious barrier around the investigation zone by contact with the epidermis (2) of said subject.

16. The apparatus as claimed in one of claims 1 to 15, additionally comprising a wired or wireless communication device (17) configured to transmit one or more measurement signals produced by the detection device to a storage or post-processing device.

17. The apparatus as claimed in one of claims 1 to 16, in which the fluidic circuit (9) is formed in an insulating support (6) and in which the electrodes of the electrochemical sensor (10) are constituted by metallic deposits on said insulating support, the metallic deposits being selected from the group consisting of silver, gold, platinum and platinum black.

18. The apparatus as claimed in one of claims 1 to 17, additionally comprising a gyroscopic module and/or at least one accelerometer for detecting a state of activity of the subject.

19. The apparatus as claimed in one of claims 1 to 18, additionally comprising a temperature sensor for measuring the temperature of the epidermis of the subject.

20. A method for determining the production (Ps) of at least one chemical species by a human or animal subject, said at least one chemical including nitric oxide NO, the method comprising:

selecting an investigation zone of an epidermis of said subject,
applying an apparatus (1) as claimed in one of claims 1 to 19 for a duration that is necessary for the production of the signal which is representative of a concentration of nitric oxide NO in the flow of sweat and the signal that is representative of a flow velocity of the flow of sweat, and
determining a measurement of the production (Ps) of nitric oxide NO by the subject from a signal that is representative of the concentration of nitric oxide NO in the flow of sweat and of a signal that is representative of the flow velocity of the flow of sweat.

21. The method as claimed in claim 20, additionally comprising:
evaluating distress in the vascular tissues of the subject from the measurement of the production of nitric oxide NO.

22. The method as claimed in claim 20, additionally comprising:
evaluating a cardiovascular capacity of the subject from the measurement of the production of nitric oxide NO.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]